# EUROPEAN PATENT APPLICATION

(11) **EP 4 781 903 A2**
(43) Date of publication of application: **29.07.2026**
(21) Application number: 26172162.5
(22) Date of filing: 19.07.2023
(51) Int. Cl.: A61B 5/01

(54) **AN IMPLANTABLE DEVICE AND A CHARGING MODULE FOR POWERING THE SAME**

(30) Priority: 22.07.2022 GB 202210781
(62) Divisional of application: 23761190.0
(71) Applicant: University of Leeds, Leeds LS2 9JT (GB)
(72) Inventor: PANDIT, Hemant, Leeds, LS2 9JT (GB); FREEAR, Steven, Leeds, LS2 9JT (GB); VAN DUREN, Bernard Hendrik, Leeds, LS2 9JT (GB); KELMERS, Edgars, Leeds, LS2 9JT (GB)
(74) Representative: HGF

(57) **Abstract**

An implantable device for monitoring an intra-articular joint environment corresponding to a joint body, for example a bone or a prosthetic joint component. The implantable device comprises: a sensor module configured to measure data indicative of at least one parameter of an intra-articular joint environment corresponding to a joint body; a communications unit operatively coupled to the sensor module and configured to transmit the measured data to an external device; and a rechargeable battery configured to power the sensor module and the communications unit, wherein the sensor module, the power supply and the communications unit are contained within a capsule, wherein the capsule is releasably mountable within an opening of the joint body, and wherein, when the capsule is releasably mounted within the opening of the joint body, the sensor module is configured to monitor the at least one parameter of the intra-articular joint environment.

## Description

This invention relates to an implantable device, in particular, but not exclusively to an implantable device with a sensor module for monitoring a joint environment, such as an intra-articular joint environment.

### BACKGROUND

Knee osteoarthritis, OA, is a problem commonly affecting the middle-aged and older population, with total knee arthroplasty (also known as total knee replacement, TKR, often being the treatment of choice for patients with severe OA and significant symptoms. Although generally successful, up to 1 in 5 patients with a TKR report "dissatisfaction with the outcome" without any obvious cause. A significant proportion of these could be avoided or caught early to improve outcomes. Similarly with hip replacements, a device warning of dangerous positions can heighten awareness, and as such, the risk of dislocation (approximately 3% of hip replacements) and/or implant failure and loosening.

With improved life expectancy and increasing preference towards leading an active life, knee replacement surgery is being increasingly performed in younger and more active patients with increasing demands put on the replaced joint for a longer duration. Artificial joints have a finite life span and fail with time, necessitating a further operation which is technically difficult, costly, and less predictable in outcome than the primary procedure.. After undergoing a TKR, patients are discharged on average after 3 days in the UK and then attend physiotherapy to optimise the knee function. Patients are seen by the orthopaedic surgeon once at 3 months after the operation and then at variable time points based upon the way a patient is progressing. However, the information obtained in these visits is just a snapshot of the patient's recovery and not a detailed evaluation of their progress since the operation. It is difficult to establish an accurate evaluation of a patient's progress without detailed information on their day-to-day activities.

Instrumented implants remain underdeveloped due to existing technology being complex, expensive, and impractical. Instrumented implants, to date, require a laboratory/controlled environment and are bulkier than standard implants, which has the disadvantage that additional bone and/or soft tissue must be removed to accommodate the instrumented implant.

In some cases, patients are provided with external tracking devices to help clinicians in monitoring a patient's progress. These are cumbersome, can introduce errors if not put on properly and require patients to remember to use them throughout the day. Therefore, the information obtained is variable and not necessarily accurate.

The present invention seeks to address at least some of these issues.

### BRIEF SUMMARY OF THE DISCLOSURE

The present disclosure provides an implantable device for monitoring an intra-articular joint environment corresponding to a joint body, for example a bone or a prosthetic joint component. The implantable device comprises: a sensor module configured to measure data indicative of at least one parameter of an intra-articular joint environment corresponding to a joint body; a communications unit operatively coupled to the sensor module and configured to transmit the measured data to an external device; and a rechargeable battery configured to power the sensor module and the communications unit, wherein the sensor module, the power supply and the communications unit are contained within a capsule, wherein the capsule is releasably mountable within an opening of the joint body, and wherein, when the capsule is releasably mounted within the opening of the joint body, the sensor module is configured to monitor the at least one parameter of the intra-articular joint environment.

This advantageously enables properties of the joint environment, such as a mechanical property, chemical property or biological property, to be monitored in real-time. The term "joint environment" as described herein includes prosthetic or native surfaces commonly used to define the synovial joint, such as articulating surfaces, as well as the synovial fluid itself. By way of example, this includes monitoring a property, such as mechanical wear, of a polymer insert typically found in replacement knee and hip joints.

The sensor module may comprise one or more of: a motion sensor, a temperature sensor, a force sensor and a chemical sensor for measuring the at least one parameter. The implantable device may comprise a non-volatile memory configured to store patient data thereon. The implantable device may comprise a support frame for mounting the sensor module in a pre-determined position within the capsule.

The sensor module may comprise a plurality of strain gauges configured to measure a force applied to the capsule from the joint body. The support frame may comprise a central region extending along a longitudinal axis of the capsule. The plurality of strain gauges may be at least partially disposed either side of the central region. The strain gauges may be arranged in a staggered manner.

The sensor module, power supply and communications unit may be embedded within the capsule. The capsule may be substantially solid. The capsule may comprise a mouldable material. Suitable mouldable materials include liquid materials and solid materials. Solid mouldable materials include powders which can be moulded into the desired shape of the capsule, for example using compression or pressure moulding. Where liquid materials, such as PMMA or polyurethane, are used, these can be cast or formed in a mould. A two-part resin is one example of a mouldable material.

The communications unit may be adapted to transmit the measured data in response to receiving a user input. The communications unit may be configured to transmit the measured data to the external device outside the joint environment.

The capsule may be releasably mountable to the joint body by a press-fit connection with the opening of the joint body. The capsule may be releasably mountable to the joint body by a locking mechanism.

The implantable device may comprise an insert for use with a prosthetic joint component. The capsule may be releasably mounted to the insert. The capsule may be mounted to the insert by a press-fit connection. The capsule may be mounted to the insert by a locking mechanism.

The insert may comprise a polymer material. The insert may comprise a recess for receiving the capsule. The insert may comprise a non-loading region adjacent a loading-region. The recess may be formed in the non-loading region.

The insert may comprise an articular surface configured to engage a corresponding articular surface of a second joint or an implant body. The loading region may be part of the articular surface.

The insert may comprise a substantially planar structure defining a normal axis. The recess may extend through the insert at an acute angle relative to the normal axis.

The insert may comprise a second articular surface spaced from the first articulating surface. The second articular surface may be configured to engage a corresponding second articular surface of the second joint body or the implant body defining the joint environment. The non-loading region may be disposed between the first and second articular surfaces.

The insert may comprise a hemi-spherical portion. The recess may be formed in an inferior aspect of the insert.

The insert may be adapted for use with any of: a replacement ankle joint, a replacement knee joint, a replacement hip joint, a replacement wrist joint, a replacement elbow joint or a replacement shoulder joint.

The implantable device may comprise a receiver circuit for receiving power wirelessly from a charging module outside the intra-articular environment and configured to charge the rechargeable battery.

There is also provided a charging module for powering an implantable device, wherein the communications unit comprises a Bluetooth Low Energy module configured to transmit the measured data in a data packet comprising the measured data and a localization parameter of the device. The charging module comprises: a receiver unit configured to receive the data packet; a charging coil configured to generate a magnetic field for wirelessly transferring power to the rechargeable battery of the implantable device, and a controller operatively coupled to the charging coil and the receiver unit, wherein the controller is configured to operate the charging coil according to a first transmission scheme, wherein the controller is configured to adapt the first transmission scheme based on the localization parameter to provide a second transmission scheme, and wherein the controller is configured to operate the charging coil according to the second transmission scheme.

The charging coil may be embedded within a fabric layer. The charging module may comprise a plurality of charging coils. A first coil of the plurality of charging coils may be arranged in a first plane. A second charging coil of the plurality of charging coils may be arranged in a second plane. The first plane may be substantially perpendicular to the second plane.

There is also provided a method of wirelessly powering an implantable device having a Bluetooth Low Energy communications unit using a charging module, wherein the charging module is configured to power the implantable device according to a first transmission scheme using one or more charging coils, the method comprising: receiving a data packet comprising a localization parameter of an implantable device, the data packet being received by a charging module from a Bluetooth Low Energy communications unit of the implantable device, adapting the first transmission scheme to a second transmission scheme based on the localization parameter, and operating the charging module according to the second transmission scheme to induce charge in the implantable device.

The method may comprise determining a performance parameter of the induced charge. The method may comprise adapting the second transmission scheme to a third transmission scheme based on the performance parameter. The method may comprise operating the charging module according to the third transmission scheme.

There is also provided a method of using an implantable device for monitoring a joint environment. Optionally, the implantable device monitors the joint environment, for example one or more mechanical properties of an insert. In some cases the implantable device monitors the joint environment between a first surgical procedure and a second surgical procedure.

There is also provided a method comprising: removing an implanted implantable device from a joint environment. In some cases, removing the implantable device comprises removing an insert into which the implantable device was mounted. The method may include mounting an implantable device within the joint environment. In some cases, the previously-removed implantable device is implanted into the joint environment. In some cases, a new implantable device is implanted into the joint environment.

### BRIEF DESCRIPTION OF THE DRAWINGS

Embodiments of the invention are further described hereinafter with reference to the accompanying drawings, in which:
Figures 1A & 1B are plan and perspective illustrations of an exemplary implantable device;
Figure 2 is a perspective view of the implantable device of Figure 1 encapsulated in a capsule;
Figure 3 illustrates the implantable device of Figure 1 mounted within an exemplary insert for use with a tibial prosthetic joint component;
Figure 4A illustrates a cross-sectional view of the implantable device of Figure 3;
Figures 4B & 4C illustrate plan and perspective views of the implantable device of Figure 3;
Figure 5A & 5B illustrate loading and non-loading regions of a tibial insert;
Figures 6A to 6H show computational model outputs illustrating different loading scenarios applied to the insert of Figures 5A & 5B;
Figures 7A to 7C show exemplary data from a section of polymer representing a tibial insert loaded under different scenarios being loaded under different scenarios;
Figure 8 shows exemplary force data measured by the implantable device of Figure 1 when mounted to an intact tibial tray;
Figure 9 shows exemplary force data measured by the implantable device of Figure 3 when mounted to a fractured tibial tray;
Figure 10 shows exemplary kinematic data measured by the implantable device of Figure 3 when mounted to a tibial tray;
Figure 11 illustrates an exemplary charging module;
Figure 12 shows exemplary charging and battery voltage data using the charging module of Figure 11;
Figure 13 illustrates an alternative exemplary charging module,
Figure 14 illustrate a further exemplary charging module;
Figures 15 & 16 illustrate exemplary methods of charging the implantable device;
Figure 17 is a schematic illustration of an exemplary acetabular cup and femoral head, and
Figure 18 illustrates an exemplary hip insert.

### DETAILED DESCRIPTION

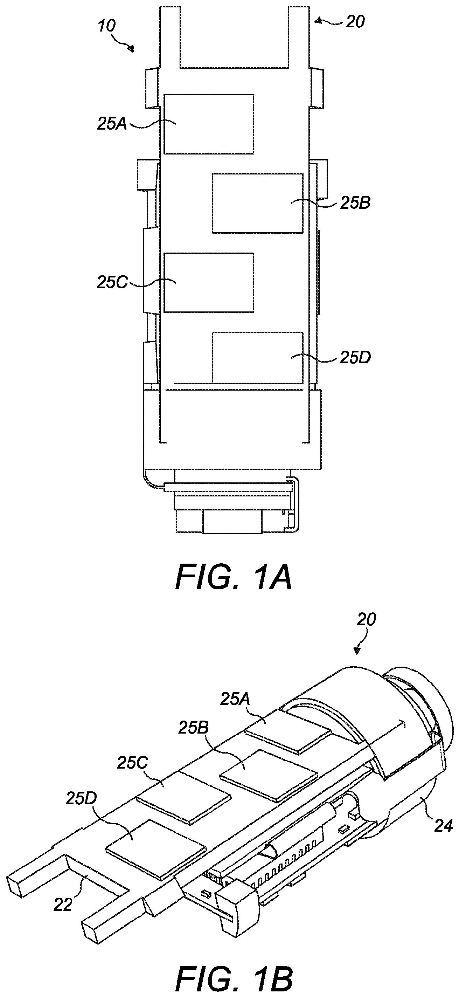

With reference to Figures 1 to 3, there is provided an implantable device 10 for monitoring an intra-articular joint environment, such as the knee joint. The illustrated implantable device 10 includes four strain gauges 25A-25D, an three-axis accelerometer and a temperature sensor for measuring force, motion and temperature within the knee joint. A communications unit, for example a Bluetooth module, is also provided to enable two-way communication with an external device, for example a smartphone of a patient, to transmit measured data an external device and, optionally, to receive software updates from the patient's smartphone or a further external device. The electrical components of the implantable device 10 are preferably encapsulated in a capsule 15 to seal the electrical components from the joint environment. By encapsulating the electrical components in this way, the implantable device 10 can be releasably mounted, for example press-fit, directly into an opening in the tibia/femur or into a prosthetic component, for example a tibial tray, as part of a tibial insert 50 if the implant is used with a total knee replacement. While a press-fit is preferable, a locking mechanism may be used additionally or alternatively to the press-fit to mount the implantable device 10 to the tibial insert 50. The locking mechanism may include any of: a resiliently deformable element, such as one or more pegs, cantilever clips, one or more fasteners, such as screws or pins. In one example, the capsule 15 comprises a threaded section for engaging a corresponding thread in the insert 50 and/or joint body.

The implantable device 10 can be releasably mounted to an alternative prosthetic component such as an acetabular cup of a replacement hip implant. As the same design of implantable device can be used with different prosthetic components, this advantageously provides a simpler manufacturing process of instrumented devices for monitoring the joint environment. It would be apparent the joint environment includes, but is not limited to, the synovial capsule and fluid contained therein as well as the surfaces defining the joint capsule, such as the natural or artificial articular surfaces. Monitoring the joint environment includes monitoring one or more properties of the joint environment using corresponding sensor(s) as explained below. Chemical, mechanical and biological properties of the joint environment are provided as examples, but it would be apparent suitable sensors would enable other properties to be measured also. By way of example, monitoring wear of the tibial insert 50 is one example of a mechanical property which the implantable device 10 can monitor.

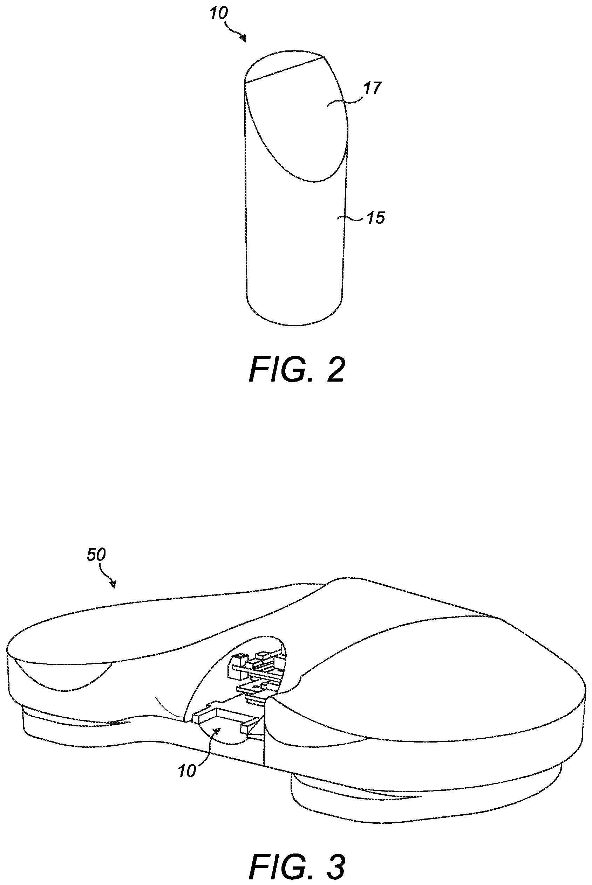

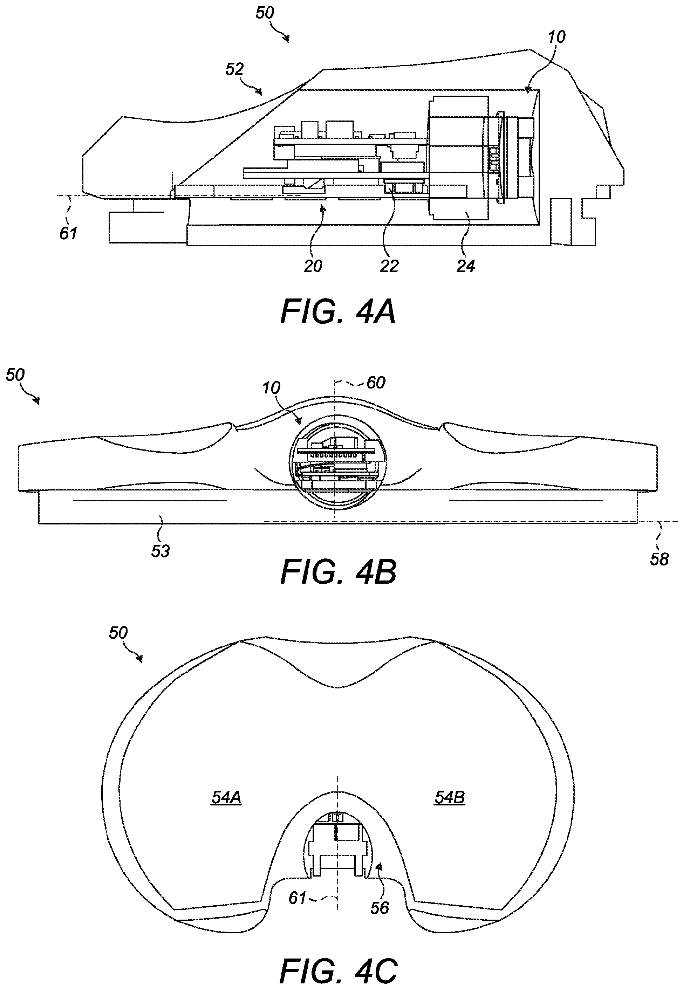

The capsule 15 encapsulating the implantable sensor 10 includes a notch 17 at one end with a profile corresponding to a notch 56 present in the tibial insert 50 (see Figure 4C). However, it would be apparent that the notch 17 is not essential and would be dependent on the position of the implantable device 10 in the tibial insert 50. Where the implantable device 10 is implanted in bones or prosthetic components of joints other than the knee (such as the hip, ankle, shoulder or elbow), it would be apparent that the notch 17 may be replaced with other suitable surface features corresponding to the joint in question, or omitted entirely. The capsule 15 has been omitted for clarity from Figures 3 & 4.

As the implantable device 10 is mounted within a tibial insert 50 of a total knee replacement, this allows for continuous monitoring of the knee joint post-surgery as data measured by the implantable device 10 can be transmitted to an external device, such as a mobile phone of the patient or surgeon. This provides better monitoring of post-operative recovery and adherence to prescribed physiotherapy exercises which helps to address the two main problem areas in primary arthroplasty: 1) rehabilitation and 2) diagnosis of implant failure. Thus, the present disclosure provides an implantable device which aids early diagnosis of infection, instability or aseptic loosening, which are three main reasons for failure, in turn providing significant benefits to the patient and reducing the diagnostic cost burden associated with multiple and expensive tests are undertaken prior to making a decision to revise an implant. A further advantage of the present implantable device 10 is that there is no additional bone loss to provide this improved monitoring, as the implantable device 10 can be introduced into existing polymer insert designs of standard thickness. In addition, the implantable device 10 can be used as a temporary device for two-stage revision of infected implants to monitor markers of infection prior to second stage procedures.

By giving patients access to their own data, the patient is given greater independence and is more able to drive and monitor their recovery as they have quantifiable feedback regarding their progress. The implantable device 10 also provides benefits to the surgeon, as they are able to continuously monitor the joint environment, compared to a snapshot in time, and can intervene in a timely manner if the data shows there is an issue developing in the joint, for example restricted range of movement, excessive wear of the tibial insert 50 preceding mechanical failure of the replacemen joint, joint infection or implant loosening. This in turn can result in fewer hospital visits, significant cost savings, early detection of a potentially catastrophic complication and timely intervention which often is less invasive, cheaper and associated with better outcomes than one that would need to have been employed if the intervention was delayed due to late diagnosis. As the implantable device 10 does not wear, the patient is able to keep the same implantable device 10 throughout their life, even if the prosthetic joint components and/or the tibial insert 50 need replacing, as the implantable device 10 can simply be removed from the old/worn component (such as the polymer tibial insert 50) and mounted into the replacement component. In some cases, it is desirable to replace the implantable device 10 when the tibial insert 50 is replaced with a new implantable device. This is particularly the case if there is an elevated risk of infection when reusing the original implantable device 10. Where a new implantable device is used, this may be the same as the original implantable device, or an improved or modified implantable device which may have different electrical components.

The present implantable device 10 also provides benefits at different time points. Intra-operative benefits include being able to measure data to improve: force distribution, joint balancing, range of motion recording, ability to choose the optimal bearing size and alignment of the joint. In the short-term (0-6 weeks): a reduced length of hospital stay, active patient involvement, early identification of poor progress, and bespoke physiotherapy input can be provided based on the measured data. In the medium-term (6 weeks - 5 years): it is possible to provide remote follow up, for example in response to patient-initiated follow up requests, follow-up based on changes in activity levels/type, and to detect other failure modes, such as infection, instability, implant loosening, and stress risers. In the long-term (> 5 years): the implantable device 10 enables better planning of revision surgery as both the component and surgical details as well as the relevant radiographs can be stored on the implantable device 10 itself, rather than requiring access to patient records which are typically stored at the hospital having originally performed the operation; diagnostic monitoring of the implantable device is also possible, for example to detect excessive wear of the tibial insert 50, implant loosening, infection; and clinical follow up remotely.

In the example shown in Figures 1 and 2, the capsule 15 is provided as a two-part polymethyl acrylate (PMMA) resin and the electrical components are embedded in the resin. However, it would be apparent that this was not essential and other compositions or arrangements of the capsule 15 can be used to seal the electrical components in the manner described. It would also be apparent that one or more of the force, temperature or motion sensing components were not essential and that one or more of these may be omitted depending on the specific requirements of the implantable device 10. It would also be apparent that in some cases additional sensors may be included in the implantable device 10, for example electro-chemical sensors for measuring chemical properties of the joint environment. Examples of electro-chemical sensors include pH sensors and glucose sensors which can be connected to I/O pins which are also suitable for connecting to the strain gauges 25A-25D. Where chemical sensors are included, the capsule 15 may include one or more channels to allow egress of intra-articular joint fluid into the capsule 15 to reach the chemical sensor. While a two-part resin is described, it would be apparent this was merely an example of a mouldable material suitable for forming the capsule 15.

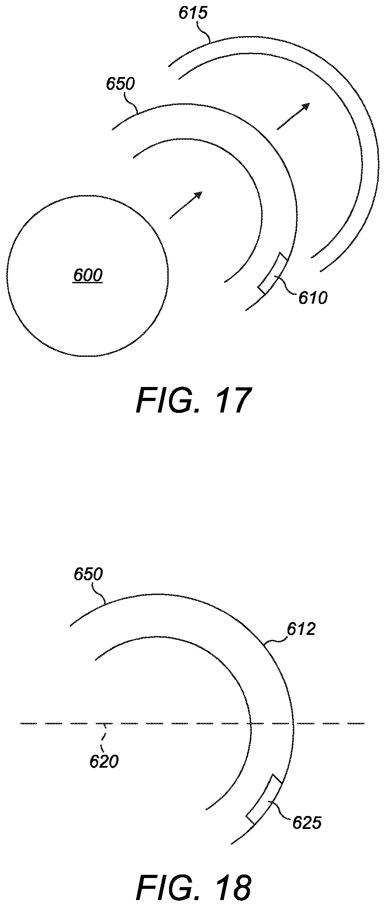

The capsule 15 defines an internal volume within which the electrical components are located. The internal volume may be partially or entirely solid. Where the capsule 15 is entirely solid (i.e. there are no hollow sections within the internal volume), a direct load path is provided between the insert 50 and the strain gauges 25A-25D, allowing the strain gauges 25A-25D to be mounted on a support frame 20 in the manner shown in Figures 1A and 1B. Where a hollow capsule 15 is used, the strain gauges 25A-25D are preferably mounted on an inner surface of the capsule 15 to detect loads applied to the capsule 15. While the capsule 15 is shown having a substantially cylindrical profile, it would be apparent this was not essential. A cylindrical profile corresponds to a round hole, such as provided in the illustrated tibial insert, but where the implantable device 10 is mounted within a recess having a non-circular profile, the capsule 15 may have a profile corresponding to the recess. This may be the case where the implantable device 10 is mounted to a hip insert 650 (see also Figures 17 and 18) which is designed to be mounted in an acetabular component 615 to form a replacement acetabular cup. The

The hip insert 650 includes a recess 625 for receiving implantable device 610 which may be arranged as described herein in relation to implantable device 10. Implantable device 610 may differ from implantable device 10 in the configuration of its capsule which may be shaped to correspond to the recess 625 in the polymer hip insert 650 which may be non-circular. This enables loads applied to the acetabular cup to be transferred to the capsule in an analogous manner as described above in relation to the tibial insert 50. The implantable device 610 may be contained within the hip insert 650, or may be partially received in the hip insert 650. Where the implantable device 610 is partially received within the hip insert 650, the recess 625 may be formed on a surface 612 configured to abut the acetabular component 615 so that the implantable device 610 is at least partially disposed between the acetabular component 615 and the hip insert 650. By positioning the recess 625 within the hip insert 650, the implantable device 610 can be located within a similar non-loading region of the acetabular cup. This can be achieved by positioning the recess on an inferior aspect of the hip insert 650. By way of example, the inferior aspect can be considered to be any part of the hip insert 650 which when considered in the anatomical position, lies inferior to a transverse plane 620 intersecting the radial centre of the hip insert 650.

In addition to the sensors described herein, the implantable device 10 preferably includes an onboard non-volatile memory for storing the measured data and patient data. By storing patient information relevant to the surgical procedure such as an operation note, radiographs, implant details, pre-operative symptoms recorded using validated measures, the patient can carry this information with them and the information will be available for any health professional without the need for asking for all the relevant information from the centre where original treatment was provided. By storing patient data on-board the implantable device 10, the patient is able to control with whom and when they share their data with, rather than relying on a central database which may not always be accessible when required. For example, the patient may have an electronic device, such as a mobile phone, which is in communication with the implantable device 10. An application on the mobile phone may allow the patient to view and share the data stored on-board the implantable device 10.

With reference to Figures 1A, 1B & 4A to 4C, the implantable device 10 includes a support frame 20 (see Figure 4A) arranged to accurately position the electrical components and sensors at a pre-determined position on the implantable device 10. The support frame 20 is preferably 3D printed to allow for greater customisation, e.g. based on the specific electrical components required, and the morphology of the joint into which the implantable device 10 will be implanted. The support frame 20 includes a first section 22 for supporting a printed circuit board having the electrical components mounted thereon, and a second section 24 arranged to position the first section 22, and therefore the electrical components, at a pre-determined position in the capsule 15. The first section 22 positions the electrical components at a pre-determined longitudinal position in the capsule 15. The second section positions the electrical components at a pre-determined radial position in the capsule 15. The support frame 20 is designed to locate the electrical components in the centre of the capsule 15. Both sides of the support frame 20 can support electrical components. A further advantage of embedding the electrical components is that the relative position between the embedded components remains fixed, improving the consistency and repeatability of the manufactured implantable device 10. One advantage of this is that any calibration parameters for the electrical components, for example the strain gauges 25A-25D, can be applied across different devices and each implantable device 10 does not need to be calibrated during the manufacturing process.

For example, the printed circuit board can be mounted on one side of the support frame 20 and the strain gauges 25A-25D can be mounted on the opposite side of the support frame 20. With reference to Figure 1, the first section 22 has a central region extending along a longitudinal axis 61 of the capsule 15. The strain gauges 25A-25D are mounted in an alternating arrangement, such that each strain gauge 25A-25D at least partially overlaps a central region of the printed circuit board, for example by intersecting the longitudinal axis 61 extending through the centre of the capsule 15 when viewed from the perspective shown in Figure 4C. It would be apparent that in some cases, the strain gauges 25A-25D may be spaced further apart relative to the longitudinal axis 61 such that one or more of the strain gauges 25A-25D do not intersect the longitudinal axis 61 (i.e. the strain gauges 25A-25D are spaced from the longitudinal axis 61 by a perpendicular distance relative to the longitudinal axis 61). This arrangement provides the ability to measure strain applied at different locations along the capsule 15 and can be used to determine the anterior-posterior and medial-lateral loads applied to the tibial insert 50 as explained below. As shown in Figure 4A, the capsule 15 has a circular cross-section and the second portion 24 circumscribes the cross-section of the capsule 15. However, it would be apparent this was not essential and that other arrangements of the second portion 24 and cross-sectional shapes of the capsule 15 can be used. In the illustrated example, the first portion 22 extends along a longitudinal axis of the capsule 15 and provides a substantially planar surface on which a printed circuit board and strain gauges 25A-25D can be secured to. The first portion 22 is connected to the second portion 24 at an end of the first portion 22. While the strain gauges 25A-25D are shown centrally mounted within the capsule 15 and distributed across the longitudinal axis 61 and a second axis perpendicular to the longitudinal axis 61 and within the plane 58, it would be apparent this was not essential and other arrangements of strain gauges distributed across two perpendicular axes (i.e. a staggered array) can be used to determine the centre of pressure of the forces applied to the capsule 15 by interpolating the measured strain data.

A recess 52 is also provided in the tibial insert 50 to receive the implantable device. The recess 52 has a cross-section which results in an interference fit between the implantable device 10 and tibial insert 50. In the illustrated embodiment the recess 52 is a blind hole, but it would be apparent this was not essential and that the recess may have a cross-section that is not circular. The tibial insert 50 has a planar surface 53 which, in use, abuts a corresponding surface on the tibial component. The planar surface 53 may define a plane 58 and a normal axis 60 relative to the plane 58. In the illustrated example, the plane 58 is approximately parallel with the coronal plane and the normal axis 60 is approximately parallel with the superior-inferior axis of the insert. The recess 52 is shown extending from a posterior side of the tibial insert 50 towards the centre of the tibial insert 50. As shown, the recess 52 extends in a direction substantially perpendicular to the normal axis 60. In the illustrated example, this is in the anterior direction, but it would be apparent this was not essential. The tibial insert 50 preferably comprises a polymer material, for example polyethylene. While a tibial insert 50 is shown, it would be apparent that inserts for use with a prosthetic component for other joints would also be suitable for use with the present implantable device 10. In some cases, the implantable device 10 may be mounted to the prosthetic component without an insert.

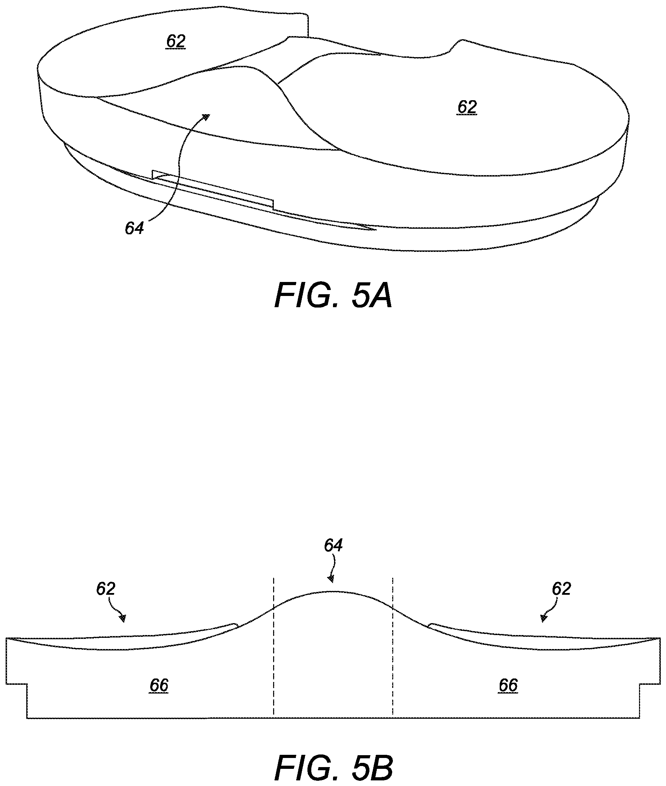

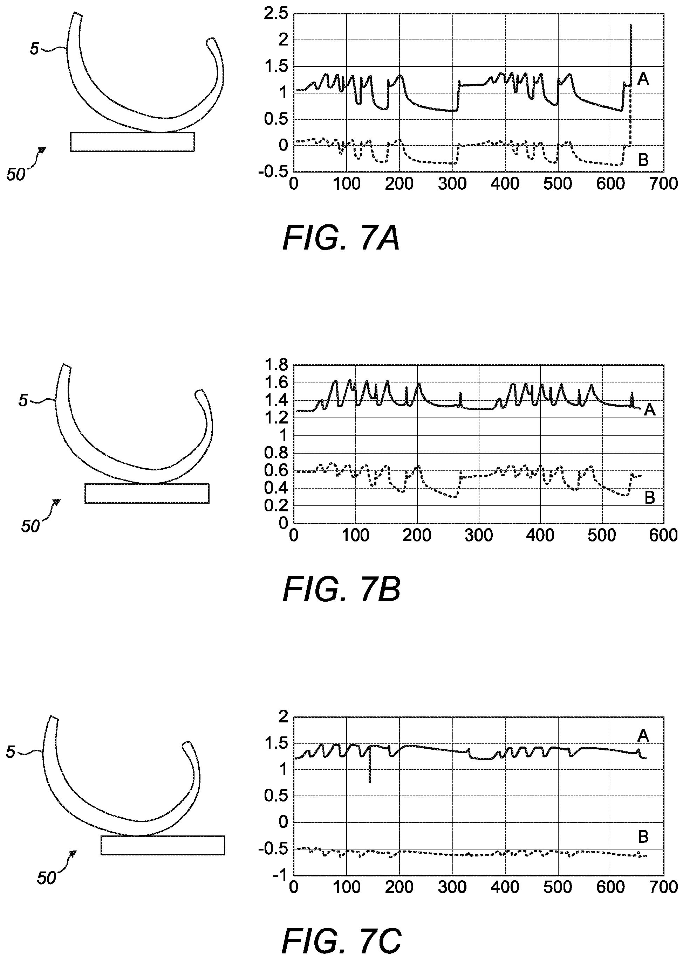

Figure 4C illustrates articular surfaces 54A, 54B of the tibial insert 50. The articular surface 54A, 54B have a profile corresponding to that of the femoral condyles (either of the native femur, or a replacement implant) such that that the replacement knee joint has a range of motion as close possible to the native range of motion. As a femoral component 5 contacts the tibial insert 50 at the articular surfaces 54A, 54B, the articular surfaces 54A, 54B can be considered a loading region 62 of the tibial insert 50 (see Figures 5A, 5B and 7A to 7C). The two articular surface 54A, 54B are spaced from one another and a non-loading region 64 is provided in the space between the articular surfaces 54A, 54B. In the example of a tibial insert 50, the non-loading region 64 corresponds to where the femoral component 5 does not contact the tibial insert 50 in normal use. It would also be apparent that as the tibial insert 50 is a continuous section of material, there will be some deformation within the non-loading region 64, but that these loads will be considerably less than the loads applied to the loading region 62 during normal activities of daily living.

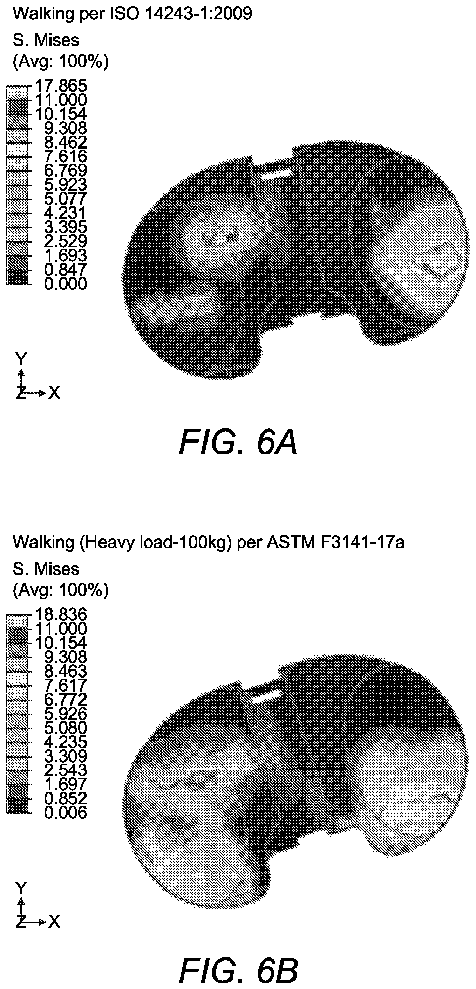

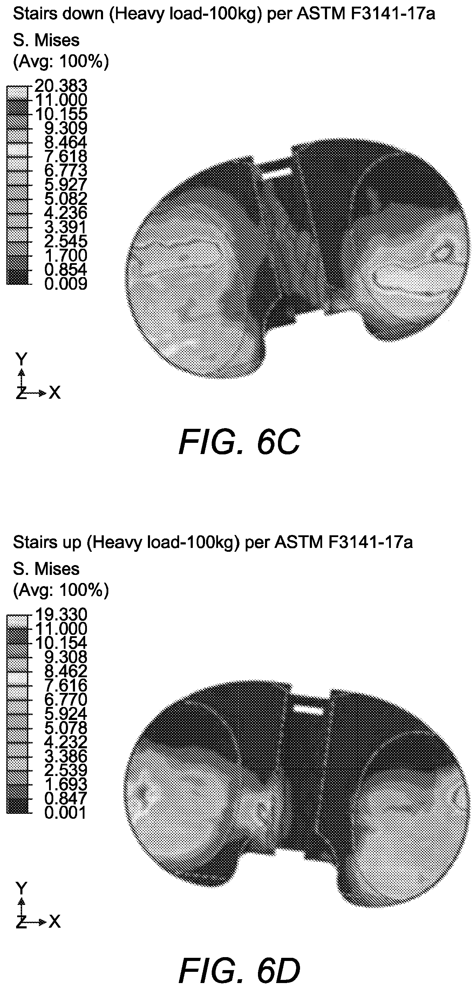

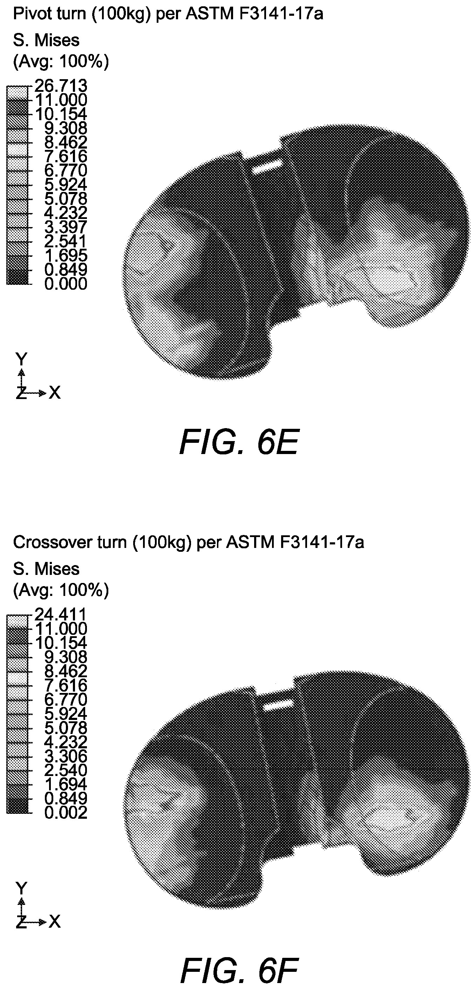

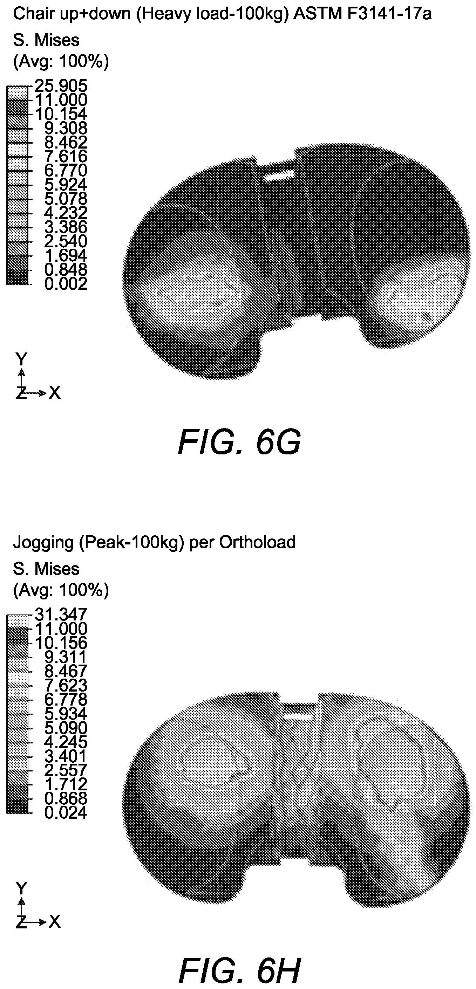

Figures 6A to 6H provide finite element analysis of exemplary loading scenarios according to ISO 14243-1:2009 and ASTM F3141-17a. These correspond to walking (Figure 6A), walking with a 100kg load (Figure 6B), walking down stairs (Figure 6C), walking up stairs (Figure 6D), performing a pivot turn (Figure 6E), performing a crossover turn (Figure 6F), getting up from and sitting down on a chair (Figure 6G) and jogging (Figure 6H). As can be seem from the computational models, while there is some strain in the inter-condylar notch (the non-loading region 64), these are significantly smaller than the loads applied to the articular surfaces 54A, 54B (the loading region 62). These are however detectable values which can be monitored using strain gauges 25A-25D or alternative force sensors e.g. capacitive sensors. Thus, while sufficient support material 66 is required under the loading region 62, it is possible to remove material in the non-loading region 64, for example to provide the recess 52 for the implantable device 10, without significantly impacting the performance of the tibial insert 50. It is therefore advantageous to provide the recess in the non-loading region 64.

By incorporating the recess 52 in the non-loading region 64, it is also considerably less complicated to obtain regulatory approval for the present implantable device 10, as its incorporation into an insert has minimal negative impact on the functionality of the insert and the overall replacement joint. By providing an implantable device 10 that can be used in a modular manner as described herein, this greatly simplifies the manufacturing process, as the implantable device 10 is a stand-alone device that can be manufactured in a controlled setting independently of the prosthetic joint component the implantable device 10 will ultimately be mounted to. Similarly, existing manufacturing processes for prosthetic joint components only require minimal modification to work with the present implantable device. For example, introducing a recess into a tibial insert 50 can be achieved by several processes, for example drilling a hole into an existing tibial insert 50, or 3D printing an insert with a recess incorporated in the original manufacturing design file.

The present implantable device 10 also has advantages regarding obtaining regulatory approval, as the implantable device can be manufactured independently of the prosthetic joint component, and therefore as the implantable device 10 is upgraded or modified, it is only the implantable device 10, rather than the entire replacement joint that must be reassessed.

As shown above, with reference to Figure 6, introducing a recess 52 into the non-loading region 64 of a tibial insert 50 has negligible impact on the performance of the tibial insert 50. By modifying an existing, already-approved, design in this way, the approval process is also streamlined as assessments regarding the existing device can be taken into account when assessing a modified tibial insert 50.

Furthermore, by encapsulating the electrical components, should there be a mechanical or electrical failure of the implantable device 10, it is possible to extract the tibial insert or the encapsulated components separately from the tibial insert 50 which can remain attached to the tibial tray (in the case of a knee joint). Thus advantageously, less invasive surgery will be required to recover the implantable device 10, as the modular components 10,50 are easier to remove/exchange than a complete tibial joint component which would be anchored to the bone. The implantable device 10 of Figure 1 has maximum diameter of 10mm diameter and a length of 25mm. However, it would be apparent that smaller dimensions (e.g. 8mm diameter) are desirable where this is possible within the constraints of the available electrical components. As a further safety measure, the recess 52, and thus the capsule 15, may be arranged to provide a minimum distance, for example 5mm, from the articular surfaces 54A, 54B.

Once the implantable device 10 of Figure 1 has been implanted, it is possible to measure its 3D orientation relative to gravity; a range of motion of the joint; gait patterns; magnitude and Centre of Pression of forces applied to the loading region 62; separate the medial-lateral loads from the anterior-posterior loads, and joint temperature.

Figures 7A to 7C show exemplary data from a section of polymer representing an alternative tibial insert loaded under different scenarios. The Y-axis shows voltages in mV, where line "A" corresponds to the strain in the right side of the tibial insert 50 and line "B" corresponds to the strain in the left side of the tibial insert 50. Figure 7A shows the sensor output when an anterior area of the tibial insert 50 is loaded by a femoral component 5 (Figure 7A, left image). Figure 7B shows the sensor output when a central area of the same tibial insert 50 is loaded by the femoral component 5 (Figure 7B, left image). Figure 7C shows the sensor output when a posterior area of the tibial insert 50 is loaded by the femoral component (Figure 7C, left image). As can be seen in the different voltage outputs (right hand images of Figures 7A to 7C), the implantable device 10 can be used to determine where load is being applied to the tibial insert 50.

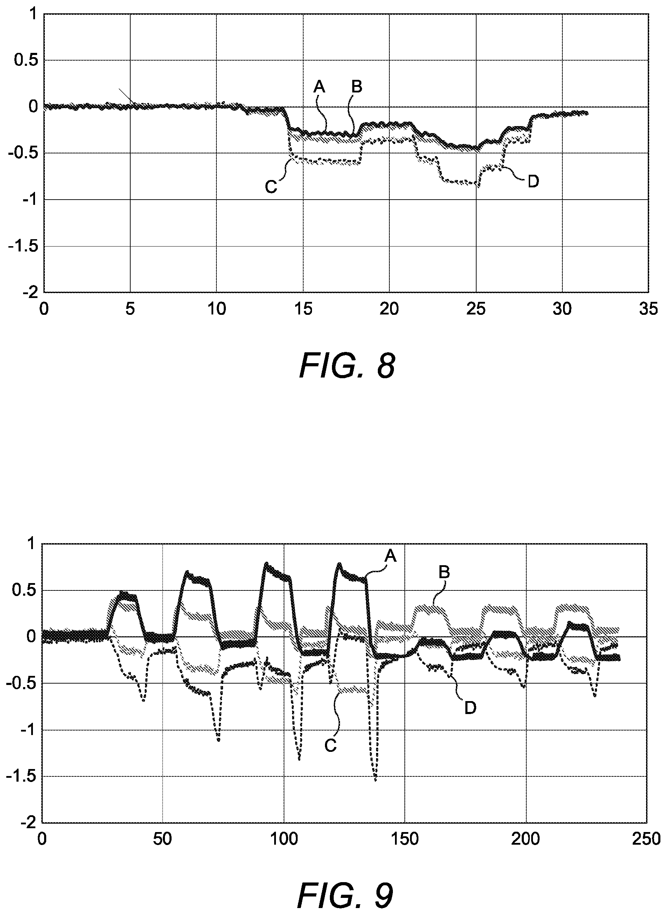

Figure 8 shows exemplary force data measured by the implantable device of Figure 3 when mounted to an intact tibial tray. The Y-axis shows voltages in mV, where lines "A", "B", "C" and "D" corresponds to the outputs from each of the four strain gauges 25A-25D of the implantable device 10. It should be noted that in Figure 8, all of the signals from the strain gauges 25A-25D are negative when loaded. However, when the tibial tray develops a fracture, the same loading conditions applied to the insert result in a different output from the force sensor. As shown in Figure 9, the output signals from the four strain gauges 25A-25D making up the force sensor are noticeably different to those of Figure 8. Specifically, some of the strain gauge outputs (notably outputs "A" and "B" in Figure 9) have changed polarity and are now positive when the tibial insert is loaded. It would be apparent that this was merely exemplary, and that in some cases a damaged tibial tray may cause the output signals to change in other ways, for example a significant change from historical values with or without a change in polarity of the signal.

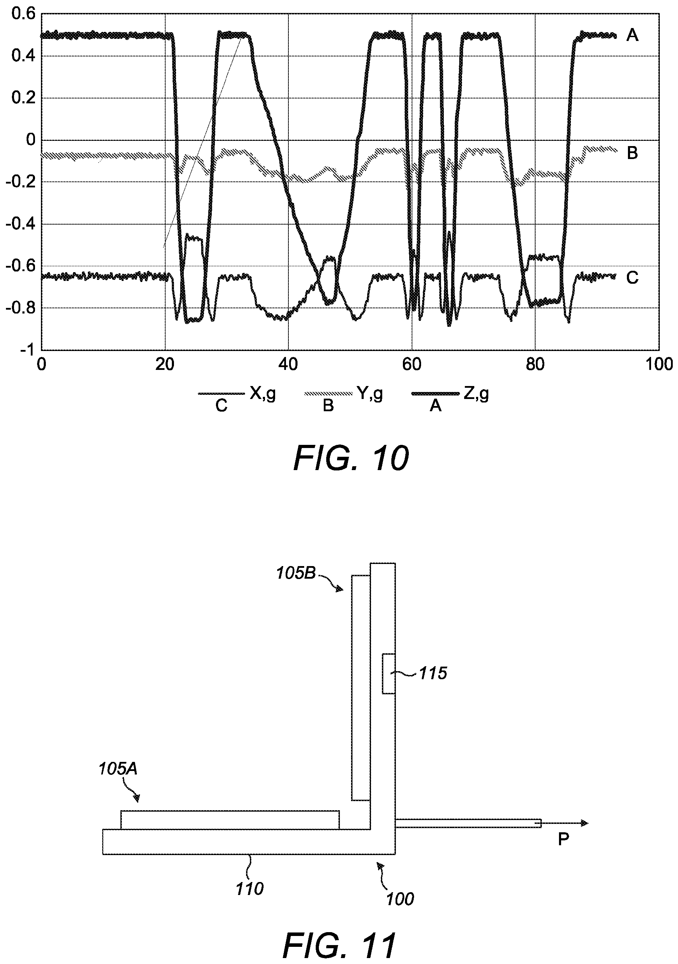

Figure 10 shows exemplary kinematic data measured by the implantable device of Figure 3 when mounted to a tibial tray. Lines "A", "B" and "C" represent the three axes of rotation of the accelerometer of the implantable device 10, and Y-axis shows the output acceleration relative to gravity. As shown by line A, the implantable device 10 can reliably measure knee flexion. Further tests have shown the implantable device 10 can also reliably measure joint kinematics during walking, running, going down stairs, going up stairs, pivoting and jumping.

Ability to monitor the intra-articular environment of a prosthetic joint, as opposed to the joint body (i.e. the bone or the tibial tray in the illustrated examples) is important as this enables clinicians to foresee implant failure. The data measured by the implantable device is better able to assist the clinical decision, as the data provided by the implantable device 10 provides a richer dataset for the clinician. For example, any combination of changes in temperature (e.g. a temperature increase relative to historical data), joint forces (e.g. a change in polarity and/or magnitude of a force measurement as described above), movement (e.g. an increase in vibrations can indicate implant loosening) may be indicative of implant failure. It would be apparent that implant failure may occur due to failure of the tibial insert 50 due to excessive wear, implant loosening and/or infection or instability. These failure modes have associated parameters which can be monitored in the manner described herein.

In some cases, the implantable device may be configured to output an alert to the external device to alert the surgeon of a change in the measured parameter(s). The implantable device 10 may have an on-board processor configured to monitor the measured data for anomalies and output the alert. The processor may store historical sensor data and use this as a reference from which to determine the current parameters are out of the expected ranges.

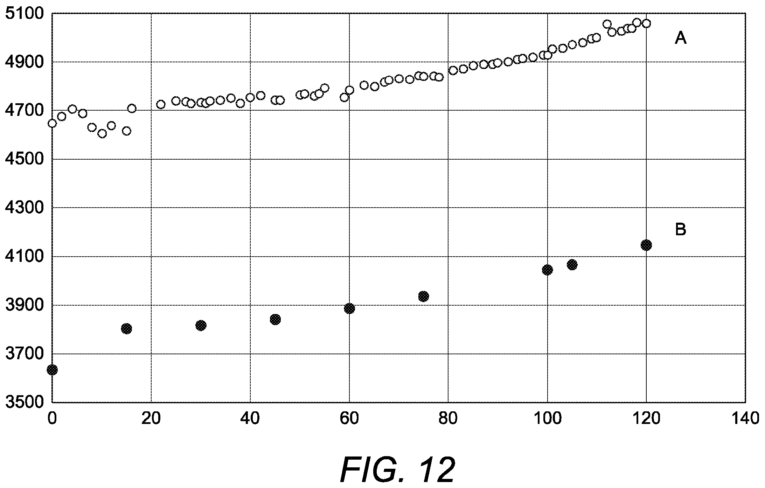

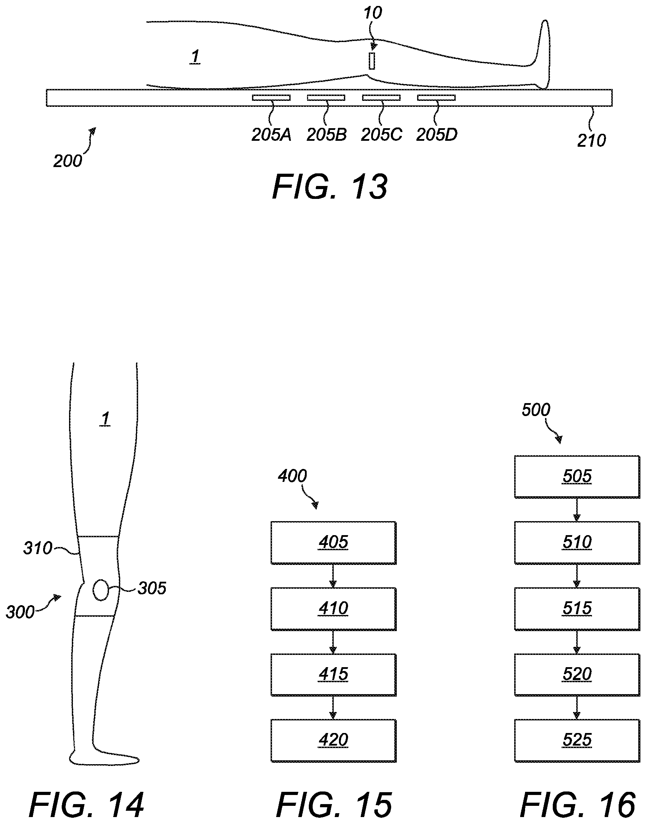

The implantable device 10 also includes a rechargeable battery, for example having a capacity of 25mAh, for powering the electrical components and a circuit for charging the rechargeable battery. With reference to Figures 11 and 12, the rechargeable battery can be charged wirelessly using a charging module 100. The charging module 100 includes two coils 105A, 105B arranged perpendicular relative to one another. A support frame 110 is provided onto which the coils 105A, 105B can be mounted. The frame can also be connected to a power source "P", such as a mains supply voltage for powering the charging module 100. In the illustrated example, the support frame 110 is rigid and holds the coils 105A, 105B in the desired perpendicular arrangement. This arrangement has been found particularly effective at inducing charge in the circuit of the implantable device when the implantable device is implanted in the body. As shown in Figure 12, the present charging module 100 can induce charge in the circuit when disposed outside the knee joint. Line "A" represents the charging voltage in mV and line "B" represents the battery voltage in mV. Tests have shown that the charging module 100 can charge the rechargeable battery at a distance of 60mm through the soft tissues around a human knee joint. The implantable device 10 includes an induction coil which can be considered a receiver coil. The charging module 100 includes an induction coil which can be considered a sender coil. However, it would be apparent that a rigid support frame 110 is not essential and the coils 105A, 105B may be attached to, for example integrated within, a sleeve or a support brace which can be worn by a patient 1 over the implantable device 10 (see Figure 13 and 14). The sleeve may be configured as a garment to be worn over the joint or maybe integrated into a flexible fabric worn which can be worn over the joint as explained below.

Figures 13 and 14 illustrate alternative charging modules. In Figure 13, the charging module 200 includes a fabric layer 210 in which multiple charging coils 205A-205D are embedded. The charging coils 205A-205D may be configured to work in the same manner as described above in relation to coils 105A, 105B. A patient 1 with an implantable device 10 is thus able to easily recharge the implantable device 10 overnight as they sleep, as the coils 205A-205D within the fabric layer 210 can be activated when the patient is lying on the fabric 210, for example in the form of a blanket or an under sheet placed under a standard bed sheet. In Figure 14, the charging module 100 is configured as a wearable knee brace 300 which includes a fabric layer 310 having multiple charging coils 305 embedded within. For example, the charging module can include a coil 305 on either side of the knee brace 300 (only one coil 305 is shown over the lateral side of the knee in Figure 14). The patient can therefore perform their daily activities while the implantable device 10 charges. It would be apparent that the implantable device 10 may transmit data as the charging module powers the implantable device 10.

The present charging module 100 includes a controller 115 configured to adapt a transmission scheme by which the coils 105A, 105B are activated to provide improved charging performance. While reference is made to the embodiment of Figure 11, it would be apparent the following method applies more broadly to charging modules and includes those illustrated in Figure 13 and 14. The charging module 100 also preferably includes a power supply or can be mains powered.

Figure 15 illustrates an exemplary method 400 of charging the implantable device 10. When the implantable device 10 transmits 405 data to the external device, this data is typically in the form of a data packet including a localization parameter corresponding to the type of communication module used to transmit the data packet. Where the communication module is a Bluetooth Low Energy module, the data packet can include data indicative of the strength of the Bluetooth signal used to transmit the data packet. The external device can then use this information to determine 410 a distance between the external device receiving the data packet and the implantable device 10, for example by calculating a received signal strength indicator (RSSI) of the data packet. As the range of the Bluetooth signal can be up to 100m in optimum conditions with an RSSI accuracy of approximately 2-4m this is sufficient to detect 415 when the patient is close to the charging module, for example blanket 200, and to activate 420 one or more transmission coils 205A-205D to recharge the implantable device 10. In one example, the controller may activate all charging coils of the blanket 200 when the implantable device 10 is detected as being in close proximity to the blanket 200. The position of the blanket 200 may be determined using steps 405 and 410 to determine its position relative to the external device. It is not essential for all charging coils to be activated as explained below.

It would be apparent that in the case of a blanket 200, there may be a large number of coils 205A-205D distributed throughout the fabric 210, and thus, the localization parameter may be used to activate one or more of charging coil(s) 205A-205D closest to the implantable device 10 in order to provide a more robust recharging process with optimised power demands as only a selection of charging coils 205A-205D are activated depending on where the patient 1 is on the blanket 300. In some cases, Bluetooth detectors located around a room in which the charging module is located can transmit the location information back to the implantable device 10. The implantable device 10 is therefore provided with its position relative to the charging module 200. As the implantable device 10 can directly monitor the rate at which it is being charged, information related to charging performance, for example charging rate of the rechargeable battery, can be combined with the location of the implantable device 10 relative to the charging module 300 to locate the implantable device 10 with a greater accuracy than would otherwise be possible using only the accuracy of Bluetooth RSSI. The transmission scheme used to operate the charging coils 205A-205D can then be adjusted accordingly to improve the coupling efficiency between the charging coils 205A-205D and the implantable device 10.

Figure 16 illustrates a second exemplary method 500 of charging the implantable device 10. For example, according to a first transmission scheme 505, each charging coil 205A-205D is sequentially activated. The charging rate of the implantable device 10 can be associated 510 with when each charging coil 205A-205D is activated. By comparing 515 the different charging rates obtained during the first transmission scheme 505, a charging coil inducing a peak charging rate is identified 520. The position of the charging coil associated with this peak charging rate, for example charging coil 205C in Figure 13, thus provides a more accurate position of the implantable device 10 (compared to relying on RSSI alone) as it is reasonable to infer that the implantable device 10 is closest to charging coil 205C. The controller 15 can then adapt the first transmission scheme such that only charging coil 205C is activated according to a second transmission scheme 525. This provides power-efficient charging as the remaining coils 205A, 205B, 205D would not be activated. However, it would be apparent that one or more additional coils (e.g. 205B and 205D) in may be activated in the second transmission scheme to account for patient movement. For example, charging coils adjacent (e.g. radially adjacent) to the charging coil identified as providing peak charging rate could also be activated.

This method 500 may be performed at multiple time points to ensure the one or more charging coil(s) nearest the implantable device 10 are activated overnight even if the patient moves in the night. This may be according to a pre-determined time interval, or in response to the charging rate dropping below a pre-determined threshold. The methods 400 and 500 may be combined in various ways. For example, instead of activating all charging coils according to step 420, the controller 115 may sequentially activate the charging coils according to step 505 without performing step 420. Alternatively, or additionally, after all of the charging coils 205A-205D have been activated according to step 420, the controller 115 may perform the steps of method 500 so that the charging coils 205A-205D are activated according to step 525 as a third transmission schedule.

By way of example, the transmission scheme used to activate the charging coils 205A-205D can be adapted by simply turning different charging coils on or off, depending on the location and/or proximity of the implantable device 10 relative to the charging coils 205A-205D. Where the charging coils are arranged perpendicular to one another, such as in Figure 11 or Figure 14, the charging coils can be run in antiphase to improve charging performance. While the phase of the charging coils can be adapted in any of the charging modules described herein, it would be apparent other parameters may be adapted to optimise charging performance and to increase the charging range. These include any combination frequency, amplitude and polarity of the charging coil signal.

The capsule can be made also from metal not only polymer material. The capsule may be a metal or polymer hollow capsule. The capsule can also have flat face, not only a hemi-spherical face. The capsule can have various shapes, including but not limited to pill, bullet, and cylindrical shapes.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.
Certain implementations are described in the following numbered clauses. The numbered paragraphs in this section are not claims. The claims are set forth below in the later section headed "claims".
Clause 1. An implantable device for monitoring an intra-articular joint environment corresponding to a joint body, for example a bone or a prosthetic joint component, the implantable device comprising:
   - a sensor module configured to measure data indicative of at least one parameter of an intra-articular joint environment corresponding to a joint body;
   - a communications unit operatively coupled to the sensor module and configured to transmit the measured data to an external device; and
   - a rechargeable battery configured to power the sensor module and the communications unit,

   wherein the sensor module, the power supply and the communications unit are contained within a capsule,
   wherein the capsule is releasably mountable within an opening of the joint body, and wherein, when the capsule is releasably mounted within the opening of the joint body, the sensor module is configured to monitor the at least one parameter of the intra-articular joint environment.
Clause 2. An implantable device according to 1, wherein the sensor module comprises one or more of: a motion sensor, a temperature sensor, a force sensor and a chemical sensor for measuring the at least one parameter.
Clause 3. An implantable device according to 1 or 2 comprising a non-volatile memory configured to store patient data thereon.
Clause 4. An implantable device according to any preceding clause comprising a support frame for mounting the sensor module in a pre-determined position within the capsule.
Clause 5. An implantable device according to any preceding clause, wherein the sensor module comprises a plurality of strain gauges configured to measure a force applied to the capsule from the joint body.
Clause 6. An implantable device according to clauses 4 and 5, wherein the support frame comprises a central region extending along a longitudinal axis of the capsule, and wherein the plurality of strain gauges are at least partially disposed either side of the central region.
Clause 7. An implantable device according to any preceding clause, wherein the sensor module, power supply and communications unit are embedded within the capsule.
Clause 8. An implantable device according to any preceding clause, wherein the communications unit is adapted to transmit the measured data in response to receiving a user input.
Clause 9. An implantable device according to any preceding clause, wherein the capsule is releasably mountable to the joint body by a press-fit connection with the opening of the joint body.
Clause 10. An implantable device according to any preceding clause comprising an insert for use with a prosthetic joint component, wherein the capsule is releasably mounted to the insert.
Clause 11. An implantable device according to 10, wherein the capsule is mounted to the insert by a press-fit connection.
Clause 12. An implantable device according to 10 or 11, wherein the insert comprises a polymer material.
Clause 13. An implantable device according to any of clause 10 to 12, wherein the insert comprises a recess for receiving the capsule.
Clause 14. An implantable device according to any of clauses 10 to 13, wherein the insert comprises a non-loading region adjacent a loading-region, and wherein the recess is formed in the non-loading region.
Clause 15. An implantable device according to any of clauses 10 to 14, wherein the insert comprises an articular surface configured to engage a corresponding articular surface of a second joint or an implant body, and wherein the loading region is part of the articular surface.
Clause 16. An implantable device according to clause 15, wherein the insert comprises a second articular surface spaced from the first articulating surface, wherein the second articular surface is configured to engage a corresponding second articular surface of the second joint body or implant body defining the joint environment, and wherein the non-loading region is disposed between the first and second articular surfaces.
Clause 17. An implantable device according to any of clauses 10 to 16, wherein the insert comprises a substantially planar structure defining a normal axis, and wherein the recess extends through the insert at an acute angle relative to the normal axis.
Clause 18. An implantable device according to any of clauses 10 to 14, wherein the insert comprises a hemi-spherical portion, and wherein the recess is formed in an inferior aspect of the insert.
Clause 19. An implantable device according to any of clauses 10 to 18, wherein the insert is adapted for use with any of: a replacement ankle joint, a replacement knee joint, a replacement hip joint, a replacement wrist joint, a replacement elbow joint or a replacement shoulder joint.
Clause 20. An implantable device according to any preceding clause, comprising a receiver circuit for receiving power wirelessly from a charging module outside the intra-articular environment and configured to charge the rechargeable battery.
Clause 21. A charging module for powering an implantable device according to any preceding clause, wherein the communications unit comprises a Bluetooth Low Energy module configured to transmit the measured data in a data packet comprising the measured data and a localization parameter of the device, the charging module comprising:
   - a receiver unit configured to receive the data packet;
   - a charging coil configured to generate a magnetic field for wirelessly transferring power to the rechargeable battery of the implantable device, and
   - a controller operatively coupled to the charging coil and the receiver unit,

   wherein the controller is configured to operate the charging coil according to a first transmission scheme,
   wherein the controller is configured to adapt the first transmission scheme based on the localization parameter to provide a second transmission scheme, and
   wherein the controller is configured to operate the charging coil according to the second transmission scheme.
Clause 22. A charging module according to clause 21, wherein the charging coil is embedded within a fabric layer.
Clause 23. A charging module according to clause 21 or 21 comprising a plurality of charging coils, wherein a first coil of the plurality of charging coils is arranged in a first plane, and a second charging coil of the plurality of charging coils is arranged in a second plane, wherein the first plane is substantially perpendicular to the second plane.
Clause 24. A method of wirelessly powering an implantable device having a Bluetooth Low Energy communications unit using a charging module, wherein the charging module is configured to power the implantable device according to a first transmission scheme using one or more charging coils, the method comprising:
   - receiving a data packet comprising a localization parameter of an implantable device, the data packet being received by a charging module from a Bluetooth Low Energy communications unit of the implantable device,
   - adapting the first transmission scheme to a second transmission scheme based on the localization parameter, and
   - operating the charging module according to the second transmission scheme to induce charge in the implantable device.
Clause 25. A method according to clause 24 comprising: determining a performance parameter of the induced charge, adapting the second transmission scheme to a third transmission scheme based on the performance parameter, and operating the charging module according to the third transmission scheme.

## Claims

1. An implantable device for monitoring an intra-articular joint environment corresponding to a joint body, for example a bone or a prosthetic joint component, the implantable device comprising:
a sensor module configured to measure data indicative of at least one parameter of an intra-articular joint environment corresponding to a joint body;
a communications unit operatively coupled to the sensor module and configured to transmit the measured data to an external device; and
a rechargeable battery configured to power the sensor module and the communications unit,
wherein the sensor module, the power supply and the communications unit are contained within a capsule,
wherein the capsule is releasably mountable within an opening of the joint body, and
wherein, when the capsule is releasably mounted within the opening of the joint body, the sensor module is configured to monitor the at least one parameter of the intra-articular joint environment.

2. An implantable device according to 1, wherein the sensor module comprises one or more of: a motion sensor, a temperature sensor, a force sensor and a chemical sensor for measuring the at least one parameter.

3. An implantable device according to 1 or 2 comprising a non-volatile memory configured to store patient data thereon.

4. An implantable device according to any preceding claim comprising a support frame for mounting the sensor module in a pre-determined position within the capsule.

5. An implantable device according to any preceding claim, wherein the sensor module comprises a plurality of strain gauges configured to measure a force applied to the capsule from the joint body, optionally and when dependent on claim 4 wherein the support frame comprises a central region extending along a longitudinal axis of the capsule, and wherein the plurality of strain gauges are at least partially disposed either side of the central region.

6. An implantable device according to any preceding claim, wherein the sensor module, power supply and communications unit are embedded within the capsule.

7. An implantable device according to any preceding claim, wherein the communications unit is adapted to transmit the measured data in response to receiving a user input.

8. An implantable device according to any preceding claim, wherein the capsule is releasably mountable to the joint body by a press-fit connection with the opening of the joint body.

9. An implantable device according to any preceding claim comprising an insert for use with a prosthetic joint component, wherein the capsule is releasably mounted to the insert, optionally wherein the capsule is mounted to the insert by a press-fit connection.

10. An implantable device according to claim 9, wherein the insert comprises a polymer material.

11. An implantable device according to claim 9 to 10, wherein the insert comprises a recess for receiving the capsule, and/or wherein the insert comprises a non-loading region adjacent a loading-region, and wherein the recess is formed in the non-loading region.

12. An implantable device according to any of claims 9 to 11, wherein the insert comprises an articular surface configured to engage a corresponding articular surface of a second joint or an implant body, and wherein the loading region is part of the articular surface, optionally wherein the insert comprises a second articular surface spaced from the first articulating surface, wherein the second articular surface is configured to engage a corresponding second articular surface of the second joint body or implant body defining the joint environment, and wherein the non-loading region is disposed between the first and second articular surfaces.

13. An implantable device according to any of claims 9 to 12, wherein the insert comprises a substantially planar structure defining a normal axis, and wherein the recess extends through the insert at an acute angle relative to the normal axis, or wherein the insert comprises a hemi-spherical portion, and wherein the recess is formed in an inferior aspect of the insert.

14. An implantable device according to any of claims 9 to 13, wherein the insert is adapted for use with any of: a replacement ankle joint, a replacement knee joint, a replacement hip joint, a replacement wrist joint, a replacement elbow joint or a replacement shoulder joint.

15. An implantable device according to any preceding claim, comprising a receiver circuit for receiving power wirelessly from a charging module outside the intra-articular environment and configured to charge the rechargeable battery.
